(19) Europäisches Patentamt
European Patent Office
Office européen des brevets

(11) Publication number : **0 646 585 A1**

(12) # EUROPEAN PATENT APPLICATION

(21) Application number : **94306404.8**

(22) Date of filing : **31.08.94**

(51) Int. Cl.[6] : **C07D 493/20,** C07C 51/353, C07C 59/01, // (C07D493/20, 319:00, 311:00, 311:00)

(30) Priority : **31.08.93 GB 9318038**

(43) Date of publication of application : **05.04.95 Bulletin 95/14**

(84) Designated Contracting States : **DE ES FR GB IT**

(71) Applicant : **Chiroscience Limited**
**Science Park,**
**Milton Road**
**Cambridge CB4 4WE (GB)**

(72) Inventor : **Ley, Steven Victor**
**12 Wordsworth Grove**
**Cambridge, CB3 9HH (GB)**
Inventor : **Woods, Martin**
**16 Philips Avenue**
**Royston, Hertfordshire, SG8 5ES (GB)**

(74) Representative : **Perry, Robert Edward**
**GILL JENNINGS & EVERY**
**Broadgate House**
**7 Eldon Street**
**London EC2M 7LH (GB)**

(54) **Adducts of bis (cyclic vinyl ethers) with carboxylic acids and their use in enantioselective synthesis.**

(57) An adduct of a 2,2'-bis(cyclic vinyl ether) such as bis-dihydropyran and a chiral compound that has the formula

$$R_1CH(XH)\text{-}COOH$$

wherein $R_1$ is an inert substituent and X is a heteroatom. Such an adduct can be used to prepare an enantiopure acid of the formula

$$R_1R_2C(XH)\text{-}COOH$$

by reaction with base and then with a corresponding electrophile capable of introducing the group $R_2$, and releasing the desired enantiomer from the appropriate diastereomer.

EP 0 646 585 A1

## Field of the Invention

This invention relates to dihydropyran derivatives, and analogues thereof, and their use.

## Background of the Invention

3,3',4,4'-Tetrahydro-6,6'-bis-2H-pyran (abbreviated herein as "bis-DHP") is known. It selectively protects 1,2-diols, to form dispiroketals, and this protection is useful for carbohydrates; see Ley et al, Tet. Lett. (1992) 33: 4767-70, and Synthesis (1992) 52-54.

EP-A-0614895 describes ring-substituted derivatives of bis-DHP, having C2 symmetry, in single enantiomer form. They are useful for the resolution of chiral 1,2-diols or related 1,2-difunctional compounds or for the desymmetrisation of prochiral 1,2-difunctional compounds, e.g. glycerol, or as catalysts.

Seebach et al, Tetrahedron 40:1313-1324 (1984), describe a synthesis of enantiopure α,α-disubstituted-α-hydroxyacids.

## Summary of the Invention

It has now been discovered that, as an example of bis-cyclic vinyl ethers joined at the respective 2-positions, bis-DHP will form dispiroketals from α-substituted carboxylic acids, and that stereo-controlled alkylation reactions can be performed on the resulting dispiroketals to give highly enantioselective syntheses of, for example, α-hydroxy acids and α-amino-acids.

Scheme 1, in which X is a heteroatom, e.g. -S- or -NZ- (wherein Z is an electron-withdrawing group such as acyl or sulphonyl) and preferably -O-, shows the relevant reactions; $R_1$ and $R_2$ are each any substituent which does not interfere with the reaction, e.g. alkyl, aryl or aralkyl optionally substituted with, say, OH, and are chosen with regard to the desired product.

Scheme 1 shows the use of bis-DHP (I) by way of example only. The same reactions can be conducted using an analogous compound which comprises two cyclic vinyl ether moieties bridged at the C atoms adjacent to the respective O atoms.

## Description of the Invention

With further reference to Scheme 1, the chirality of compound II ($R_1 \neq H$) determines the stereochemistry of the dispiroketal (III) framework. Following treatment with base, alkylation with $R_2$-Y (an electrophile) then takes place to introduce a new alkyl group ($R_2$) on the equatorial face, and throughout the reactions there is a preservation of chirality; by this means, for example, (S)-lactic acid may be converted into (S)-2-hydroxy-2-methylpentanoic acid, or (S)-phenylalanine converted into (R)-α-methylphenylalanine. When carrying out the process on amino-acids, it may be necessary to stabilise the dispiroketal by use of a protecting group on the amino function of the amino acid (X = -NZ-).

Schemes 2 and 3 show examples of the invention, i.e. steps **III** → IV → V in Scheme 1 wherein $R_1$ = Me and (in Scheme 2) $R_2$ = R-CHOH-. R can be derived from an aldehyde or symmetrical, e.g. cyclic, ketone, e.g. RCHO or RCOR or another reactant which will undergo the aldol reaction; R may be, for example, Ph or vinyl. In Scheme 3, $R_2$ may be, for example, benzyl or ethyl. Y is a radical that promotes alkylation with $R_2$; examples of suitable nucleofuges are well known, and include Br.

By way of example, in connection with α-hydroxyacids at least, the present invention provides a reaction with a homochiral substrate which gives a majority of one diastereomer, with the substituent on the hydroxy acid preferentially adopting an equatorial position (see Scheme 1; X = -O-). This tendency, in conjunction with maximisation of anomeric stabilisation, controls the configuration at the spiroketal carbons. Having stored the chiral information as the dispiroketal, the original stereogenic centre may be destroyed by deprotonation to give the enolate which may then undergo a diasteroselective reaction with an electrophile. Finally, deprotection affords the product of a useful overall enantioselective transformation.

In a specific embodiment of the invention (see Scheme 4), (S)-lactic acid **2** was protected by acid-catalysed reaction with the bis-dihydropyran **1**, giving a mixture of diastereoisomers **3** and **4**. The absence of water is required to avoid the rapid decomposition of the bis-dihydropyran to a mixture of the enone **5** and spiroketone **6**. Optimisation of the reaction conditions for the preparation of the lactic acid derivative indicated that a strong acid such as HCl (used as a 1.0 M solution in ether) is preferred for efficient conversion. Toluene was found to be a superior solvent to chloroform and tetrahydrofuran (THF). In conjunction with relatively low temperatures (up to room temperature, e.g. 0°C), these conditions gave both the best yield and highest ratio of diastereoisomers.

The major diastereoisomer **3** can be obtained free from the minor compound **4** by recrystallisation, with a single recrystallisation from 40-60 petrol sufficing to remove all traces by 250 MHz $^1H$ NMR. The structure of the major compound was determined by X-ray crystallography, and found to have an all-chair conformation, with the substituent equatorial and the maximum anomeric stabilisation. The dioxane ring is somewhat flattened due to the presence of the carbonyl group. The structure of the minor diastereoisomer should represent a thermodynamically less stable compromise between an all-chair conformation having an unfavourable 1,3-diaxial interaction between the substituent and a carbon-oxygen bond, and a structure possessing a twist boat conformation for the dioxane ring which relieves this steric interaction at the expense of eclipsing strain and reduced anomeric stabilisation.

Deprotonation of **3** occurred readily on treatment with lithium diisopropylamide (LDA) in THF at -78 °C. The enolate thus generated reacted in a highly stereoselective manner with allyl and benzyl bromide, and in a less selective manner with n-alkyl iodides, to give predominantly the enantiomer shown in Scheme 3; see Table 1. The particular products **7** (and opposite diastereomers **8**) are identified in the Examples.

N,N'-Dimethylpropyleneurea (DMPU) was added to the lithium enolates to give greater reactivity. In the cases where moderate selectivity was observed, this could be improved by the use of potassium hexamethyldisilazide (KHMDS) instead of LDA. As has been reported by Seebach, in Modern Synthetic Methods, ed. Scheffold, Springer-Verlag (1986), pages 125-257, the deprotonation of the diisopropylamine generated during enolate formation by an equivalent of n-butyllithium gives increased yields.

When the lithium enolate of **3** reacted with carbonyl compounds, as shown in Scheme 2, excellent diastereoselectivity was observed (see Table 2). In most cases, only one of the four possible diastereoisomers was produced; the particular products **9** are identified in the Examples. Even in the reaction with acetaldehyde, which might have been predicted to give the lowest level of stereoselectivity, a surprising 93:4 ratio (based on material isolated) of two separable diastereoisomeric products was obtained. In the reactions with aldehydes, high yields were consistently obtained; reactions with ketones gave lower yields, although excellent selectivity as maintained.

Table 1 (Scheme 3)

| $R_2$-Y | Base | Diastereomer Ratio | Yield (%)[a] |
|---|---|---|---|
| benzyl bromide | LDA | >98:2[b] | 72 |
| benzyl bromide | LDA[c] | >98:2[b] | 86 |
| allyl bromide | LDA | 96:4[d] | 95(71[e]) |
| allyl bromide | LDA[c] | 96:4[d] | 94 |
| ethyl iodide | LDA | 81:19[d] | 83 |
| ethyl iodide | LDA[c] | 82:18[d] | 84(67,15[f]) |
| ethyl iodide | KHMDS | 89:11[d] | 75 |
| n-propyl iodide | LDA | 77:23[d] | 73 |
| n-propyl iodide | LDA[c] | 83:17[d] | 79(60,13[f]) |

a) Yield refers to the mixture of diastereoisomers isolated by flash chromatography. Yields in parameters refer to yields of single diastereoisomers obtained by further purification techniques.
b) The minor diastereoisomer could not be detected by 400 MHz $^1$H or 62.5 MHz $^{13}$C NMR.
c) 1.1 eq. n-BuLi solution added after enolate formation.
d) Determined by capillary GC.
e) Recrystallised from MeOH.
f) Preparative HPLC separation.

Table 2 (Scheme 2)

| Electrophile | Yield (%)[a] |
|---|---|
| benzaldehyde | 96 |
| acrolein | 94 |
| acetaldehyde | 93 |
| cyclopentanone | 35 (46) |
| acetophenone | 29 (56) |

a) Yield of recovered starting material in parentheses. This was typically a 1:1 mixture of **3** and its C15 epimer, as a result of non-selective quenching of the enolate.

In each case, the product **9** was a single diastereoisomer by 400 MHz $^1$H and 100 or 62.5 MHz $^{13}$C NMR.

The following Examples illustrate the invention.

Example 1

**(6*R*,7*R*,15*S*)-15-methyl-1,8,13,16-tetraoxadispiro[5.0.5.4]-hexadecan-14-one, 3.**

A mixture of *bis*-dihydropyran 1 (3.41g, 2.1 mmol) and lactic acid 2[14] (1.85g, 1 eq.) were dried *in vacuo* for 1 hour to remove traces of water. The mixture was placed under argon, and toluene (25ml) and a 1.0 M solution of HCl in ether (2.0 ml, 10 mol%) were added. The mixture was stirred at room temperature for 48 hrs during which time the mixture became dark, then the solvent was evaporated *in vacuo* and the residue purified by flash chromatography (20% ether/petrol). This gave a mixture of **3** and **4** in a combined yield of 85% and 12:1 ratio by $^1$H nmr. A single recrystallisation from 40-60 petrol (15ml/g) gave diastereoisomerically pure **3**. MPt. 84-86°C with prior softening (40-60 petrol). $^1$H NMR ($CDCl_3$, 200 MHz) δ 4.31 (1H, q, J=7.0 Hz, H15), 4.00-3.54 (4H, m, 2×H2, 2×H9), 2.01-1.41 (15H, m, inc. 3H, d, J=7.0 Hz, Me; 6×$CH_2$); $^{13}$C NMR ($CDCl_3$, 50 MHz) δ 170.6 (C=O), 103.3, 95.4 (C6, C7), 65.8 (C15), 62.7, 62.3 (C2, C9), 28.4, 27.9 (2×$CH_2$), 24.6 ($CH_3$), 24.4, 18.2, 18.0, 17.2 (4×$CH_2$); IR (Nujol mull) 1740 cm$^{-1}$ (C=O); MS (EI) m/z 257.1 ($MH^+$), 168.1, 156.1, 128.1, 111.1. Analysis calculated for $C_{13}H_{20}O_5$: C 60.92, H 7.86. Found C 61.13, H 8.02. $[\alpha]_D^{25}$ -124 (c=0.96, $CHCl_3$).

***General procedure for enolate alkylations.*** A solution of LDA in THF (2.5 ml) was prepared under argon from diisopropylamine (1.1 eq.) and n-BuLi solution (1.6 M solution in hexanes, 1.1 eq). After stirring for 20 minutes and cooling to -78°C, a solution of **3** in THF (1.5 ml) was added *via* cannula, the flask being rinsed with THF (2×0.5 ml). Where deprotonation of the diisopropylamine generated by this procedure was required, a second portion of n-BuLi solution (1.1 eq.) was added after 30 minutes had elapsed. DMPU (0.5 ml) was added and the mixture allowed to stir for 30 minutes, then the appropriate electrophile (2 eq.) was introduced. The reactions were monitored by tlc until no further change was observed then the mixture was allowed to warm to room temperature and quenched with 80% saturated ammonium chloride solution. The mixture was extracted with ether (×3), the combined organic phases dried ($MgSO_4$), evaporated *in vacuo* and the residue purified by flash chromatography on silica to give the desired compound.

Example 2

**(6*R*,7*R*,15*S*)-15-benzyl-15-methyl-1,8,13,16-tetraoxadispiro[5.0.5.4]-hexadecan-14-one, 7a.**

By the general procedure described above using **3** (121 mg, 0.47 mmol) and benzyl bromide (124 μl, 2.2 eq.), **7a** was obtained (141 mg, 86%) after flash chromatography (15% ether/petrol) as a colourless oil. $^1$H NMR ($CDCl_3$, 400 MHz) δ 7.25-7.15 (5H, m, Ar*H*), 3.79-3.69 (1H, m), 3.63-3.56 (2H, m), 3.44-3.37 (1H, m, 2×H2, 2×H9), 3.23 (1H, d, J=13.2 Hz, PhC*H*$_2$), 2.85 (1H, d, 1=13.2 Hz, PhC*H*$_2$), 1.99-1.43 (15H, m, inc. 1.46, 3H, s, Me; 6×$CH_2$); $^{13}$C NMR ($CDCl_3$, 62.5 MHz) δ 172.8 (C=O), 136.1 (*ipso*-C), 131.2, 127.6 (*ortho/meta*-C), 126.5 (*para*-C), 103.7, 95.7 (C6, C7), 76.0 (C15), 62.0, 61.8 (C2, C9), 46.6 ($PhCH_2$), 29.1, 28.6 (2×$CH_2$), 26.3 (Me), 25.0, 24.3, 18.2, 17.5 (4×$CH_2$); IR (thin film) 2949, 1746, 1454, 1368, 1273, 1214 cm$^{-1}$; MS (EI) m/z 347.2 ($MH^+$), 255.2, 185.1, 167.1, 146.1, 118.1. Analysis calculated for $C_{20}H_{26}O_5$: C 69.35, H 7.56. Found C 69.34, H 7.45. $[\alpha]_D^{25}$ -89.7 (c=1.0, $CHCl_3$).

Example 3

**(6*R*,7*R*,15*S*)-15-(2-propenyl)-15-methyl-1,8,13,16-tetraoxadispiro[5.0.5.4]-hexadecan-14-one, 7b.**

By the general procedure using **3** (152 mg, 0.59 mmol) and allyl bromide (103 μl, 2.0 eq.), **7b** was obtained (166 mg, 94%) after flash chromatograpby (15% ether/petrol) as a 96:4 mixture of diastereoisomers by GC (170°C). Recrystallisation from methanol gave the pure major diastereoisomer, MPt. 74.5-76.5°C. $^1$H NMR ($CDCl_3$, 400 MHz) δ 5.91-5.80 (1H, m,H2'), 5.05 (1H, s, H3'-*cis* to H2'), 5.02 (1H, d, J=5.5 Hz, H3'-*trans* to H2'), 3.89-3.76 (2H, m), 3.74-3.69 (1H, m), 3.65-3.60 (1H, m, 2×H2, 2×H9), 2.55 (1H, dd, J=13.6, 7.3 Hz), 2.43 (1H, dd, J=13.6, 7.3 Hz, 2×H1'), 1.99-1.87 (2H, m), 1.80-1.70 (2H, m), 1.67-1.42 (11H, m, inc. 1.46, 3H, s, Me; 6×$CH_2$); $^{13}$C NMR ($CDCl_3$, 100 MHz) δ 172.5 (C=O), 132.9 (C2'), 118.1 (C3'), 103.8, 95.7 (C6, C7), 75.7 (C15), 62.1, 61.9 (C2, C9), 45.6 (C1'), 29.0, 28.7 (2×$CH_2$), 26.1 (Me), 25.0, 24.4, 18.2, 17.5 (4×$CH_2$); IR (thin film) 2950, 2884, 1747, 1640, 1214, 1076 cm$^{-1}$; MS (CI) m/z 297.2 ($MH^+$), 255.1, 168.1, 111.1. Analysis calculated for $C_{16}H_{24}O_5$: C 64.85, H 8.16. Found C 64.94, H 8.39. $[\alpha]_D^{25}$ -75.6 (c=1.02, $CHCl_3$).

Example 4

**(6*R*,7*R*,15*S*)-15-ethyl-15-methyl-1,8,13,16-tetraoxadispiro[5.0.5.4]-hexadecan-14-one, 7c and**

**(6*R*,7*R*,15*R*)-15-ethyl-15-methyl-1,8,13,16-tetraoxadispiro[5.0.5.4]-hexadecan-14-one, 8c**

By the general procedure using **3** (129 mg, 0.50 mmol) and ethyl iodide (81 µl, 2.0 eq.), a mixture of **7c** and **8c** were obtained in a ratio of 82:18 by GC of the crude product (160°C). Flash chromatography (10% ether/petrol) followed by HPLC separation gave **7c** (96 mg, 67%) and **8c** (22.2 mg, 15.5%).

Major diastereoisomer **7c**: MPt. 79-81°C without recrystallisation. $^1H$ NMR ($CDCl_3$, 400 MHz) δ 3.92-3.77 (2H, m), 3.75-3.69 (1H, m), 3.67-3.61 (1H, m, 2×H2, 2×H9), 2.01-1.47 (17H, m, inc. 1.51, 3H, s, Me, 7×$CH_2$), 0.92 (3H, t, J=7.5 Hz, Me2'); $^{13}C$ NMR ($CDCl_3$, 62.5 MHz) δ 173.1 (C=O), 103.8, 95.5 (C6, C7), 76.2 (C15), 62.1, 61.9 (C2, C9), 34.5, 29.1, 28.7 (3×$CH_2$), 26.1 (Me), 25.1, 24.4, 18.3, 17.5 (4×$CH_2$), 8.1 (C2'); IR (thin film) 2950, 1746, 1456, 1368, 1272, 1168, 1076, 980 $cm^{-1}$; MS (EI) m/z 285.2 ($MH^+$), 255.1, 226.1, 184.1, 168.1, 111.1, 98.1. Analysis calculated for $C_{15}H_{24}O_5$: C 63.36, H 8.50. Found C 63.43, H 8.73. $[\alpha]_D^{30}$ -87.3 (c=1.0, $CHCl_3$).

Minor diastereoisomer **8c:** MPt. 81-86°C without recrystallisation. $^1H$ NMR ($CDCl_3$, 400 MHz) δ 3.97-3.87 (1H, m), 3.82-3.64 (3H, m, 2×H2, 2×H9), 2.03-1.91 (3H, m), 1.88-1.73 (3H, m), 1.71-1.47 (8H, m, 7×$CH_2$), 1.43 (3H, s, Me), 1.05 (3H, t, J=7.5 Hz, Me2'); $^{13}C$ NMR ($CDCl_3$, 62.5 MHz) δ 173.8 (C=O), 103.3, 95.7 (C6, C7), 75.2 (C15), 62.3, 62.1 (C2, C9), 31.7, 29.3, 28.7, 25.2, 24.45 (5×$CH_2$), 24.38 (Me), 18.4, 17.5 (2×$CH_2$), 6.9 (C2'); IR (thin film) 2961, 1736, 1442, 1367, 1276, 1159, 1101, 1031, 978 $cm^{-1}$; MS (EI) m/z 285.0 ($MH^+$), 185.0, 168.0, 127.0, 111.0. Accurate mass calc. for $C_{15}H_{25}O_5$ ($MH^+$); 285.1702. Found 285.1716. $[\alpha]_D^{30}$ -62.7 (c=1.0, $CHCl_3$).

**Preparation of 7c/8c using KHMDS as base.**

To a solution of **3** (132 mg, 0.52 mmol) in THF (5 ml) under argon at -78°C was added a 0.5 M solution of KHMDS in toluene (1.34 ml, 1.3 eq.). After 20 minutes, ethyl iodide (82 µl, 2.0 eq.) was added and the reaction stirred at -78°C for 2 hours, then allowed to warm to room temperature over 2 hours. The mixture was poured into 80% saturated ammonium chloride solution, which was then extracted with ether (×4). The organic extracts were dried and evaporated *in vacuo* and the residue purified by flash chromatography (10% ether/petrol) to give a mixture of **7c** and **8c** (110 mg, 75%). Spectral data as given above. Ratio of **7c**:**8c** determined by GC to be 89:11.

Example 5

**(6*R*,7*R*,15*S*)-15-propyl-15-methyl-1,8,13,16-tetraoxadispiro[5.0.5.4]-hexadecan-14-one, 7d and (6*R*,7*R*,15*R*)-15-propyl-15-methyl-1,8,13,16-tetraoxadispiro[5.0.5.4]-hexpdecan-14-one, 8d.**

By the general procedure using **3** (147 mg, 0.57 mmol) and n-propyl iodide (112 µl, 2.0 eq.), a mixture of **7d** and **8d** were obtained in a ratio of 83:17 by GC of the crude product (170°C). Flash chromatography (10% ether/petrol) followed by HPLC separation gave **7d** (90 mg, 60%) and **8d** (18 mg, 13%).

Major diastereoisomer: MPt. 59-61°C (pentane). $^1H$ NMR ($CDCl_3$, 400 MHz) δ 3.92-3.80 (2H, m), 3.76-3.72 (1H, m), 3.67-3.63 (1H, m, 2×H2, 2×H9), 2.03-1.90 (2H, m), 1.85-1.47 (16H, m, inc. 1.51, 3H, s, Me), 1.39-1.26 (1H, m, 8×$CH_2$), 0.88 (3H, t, J=7.4 Hz, Me3'); $^{13}C$ NMR ($CDCl_3$, 250 MHz) δ 173.3 (C=O), 103.9, 95.6 (C6, C7), 76.1 (C15), 62.2, 62.0 (C2, C9), 43.9 (C1'), 29.2, 28.8 (2×$CH_2$), 26.8 (Me), 25.2, 24.5, 18.4, 17.6, 17.0 (5×$CH_2$), 14.4 (C3'); IR (thin film) 2954, 1746, 1443, 1355, 1273, 1196, 1076 $cm^{-1}$; MS (EI) m/z 299.2 ($MH^+$), 255.1, 198.1, 185.1, 168.1, 111.1, 98.1. Analysis calculated for $C_{16}H_{26}O_5$: C 64.41, H 8.78. Found C 64.38, H 8.90. $[\alpha]_D^{26}$ -76.9 (c=1.0, $CHCl_3$).

Minor diastereoisomer: MPt. 71.5-78°C without recrystallisation. $^1H$ NMR ($CDCl_3$, 400 MHz) δ 3.95-3.86 (1H, m), 3.81-3.77 (1H, m), 3.71-3.64 (2H, m, 2×H2, 2×H9), 2.02-1.89 (3H, m), 1.83-1.48 (13H, m, 8×$CH_2$), 1.44 (3H, s, Me), 0.95 (3H, t, J=7.3 Hz, Me3'); IR (thin film) 2940, 1738, 1444, 1367, 1276, 1200, 1030 $cm^{-1}$; MS (EI) m/z 299.2 ($MH^+$), 198.1, 185.1, 168.1, 111.1, 98.1. Accurate mass calculated for $C_{16}H_{27}O_5$ ($MH^+$): 299.1858. Found 299.1855. $[\alpha]_D^{25}$ -67.5 (c=1.0, $CHCl_3$).

**Preparation of 7d/8d using KHMDS as base.**

To a solution of **3** (156 mg, 0.61 mmol) in THF (2.5 ml) under argon at -78°C was added a 0.5 M solution of KHMDS in toluene (1.58 ml, 1.3 eq.). After 45 minutes, n-propyl iodide (119 µl, 2.0 eq.) was added and the reaction stirred at -78°C for 2 hours, then allowed to warm slowly to room temperature. The mixture was poured into 80% saturated ammonium chloride solution, which was then extracted with ether (×3). The organic extracts were dried and evaporated *in vacuo* and the residue purified by flash chromatography (10% ether/hexane) to give a mixture of **7d** and **8d** (121 mg, 67%). Spectral data as given above. Ratio of **7d:8d** determined by GC to be 92:8.

***General procedure for aldol reactions.*** The enolate of **3** was prepared in the same way as for the alkylation reactions above. A second equivalent of n-BuLi solution was added in all cases except that of benzaldehyde. After addition of the electrophile the reaction was followed by tlc, and when it was judged to be complete the reaction was quenched at -78°C by the addition of saturated ammonium chloride solution. The mixture was then allowed to warm to room temperature, water added to dissolve precipitated salts and extraction carried out as for the alkylation reactions.

Example 6

**[6*R*,7*R*,15*S*,(*S*)]-15-[(hydroxy)phenylmethyl]-15-methyl-1,8,13,16-tetraoxadispiro[5.0.5.4]-hexadecan-14-one, 9a.**

By the general procedure, without a second equivalent of n-BuLi solution, using **3** (256 mg, 1 mmol) and benzaldehyde (freshly distilled, 152 μl, 1.5 eq.), compound **9a** was obtained (349 mg, 96%) after flash chromatography (two columns, 50% ether/petrol and 33% ether/petrol) as a white foam. MPt. 46-49°C (pentane). $^1$H NMR ($CDCl_3$, 400 MHz) 7.38-7.25 (5H, m, ArH), 4.84 (1H, s, PhC*H*), 4.37 (1H, br. s, OH), 4.06-3.95 (1H, m, H9-*ax*,), 3.85-3.75 (2H, m, H2-*ax*, H9-*eq*), 3.71-3.65 (1H, m, H2-*eq*), 2.07-1.90 (3H, m), 1.79-1.49 (9H, m, 6×$CH_2$), 1.40 (3H, s, Me); $^{13}$C NMR ($CDCl_3$, 62.5 MHz) δ 169.2 (C=O), 137.6 (*ipso*-C), 128.5, 126.9 (*ortho/meta*-C), 127.6 (*para*-C), 103.6, 95.6 (C6, C7), 79.4, 79.1 (PhC, C15), 62.5, 62.0 (C2, C9), 28.5, 28.4, 24.4, 23.8 (4×$CH_2$), 23.0 (Me), 17.8, 17.0 (2×$CH_2$); IR (thin film) 3465, 2951, 1750, 1453, 1369, 1213, 1073 $cm^{-1}$; MS (EI) m/z 363.2 ($MH^+$), 256.1, 185.1, 168.1, 111.1, 98.1. Analysis calculated for $C_{20}H_{26}O_6$: C 66.28, H 7.23. Found C 66.17, H 7.24. $[\alpha]_D^{28}$ -59.5 (c=1.0, $CHCl_3$).

Example 7

**[6*R*,7*R*,15*S*,(*S*)]-15-(1-hydroxy-2-propenyl)-15-methyl-1,8,13,16-tetraoxadispiro[5.0.5.4]-hexadecan-14-one, 9b.**

By the standard procedure using **3** (125 mg, 0.49 mmol) and acrolein (82 μl, 2.5 eq.), compound **9b** was obtained (145 mg, 94%) after flash chromatography (25% ether/petrol). MPt. 83-85°C (ether/pentane). $^1$H NMR ($CDCl_3$, 400 MHz) δ 5.97 (1H, ddd, J=17.2, 10.3, 7.6 Hz, H2'), 5.33 (1H, ddd, 17.2, 1.6, 1.1 Hz, H3'-*trans* to H2'), 5.27 (1H, dd, 10.2, 1.5 Hz, H3'-*cis* to H2'), 4.15 (1H, s, OH), 4.10 (1H, d, J=7.6 Hz, H1'), 4.03-3.93 (1H, m), 3.87-3.68 (3H, m 2×H2, 2×H9), 2.05-1.50 (12H, m, 6×$CH_2$), 1.46 (3H, s, Me); $^{13}$C NMR ($CDCl_3$, 62.5 MHz) δ 169.4 (C=O), 134.7 (C2'), 119.2 (C3'), 103.8, 95.9 (C6, C7), 80.0 (C1'), 79.9 (C15), 62.9, 62.4 (2×$CH_2O$), 28.8, 28.7, 24.8, 24.2 (4×$CH_2$), 23.8 (Me), 18.2, 17.3 (2×$CH_2$); IR (thin film) 3472, 2951, 1748, 1443, 1371, 1241, 1073, 1002 $cm^{-1}$; MS (EI) m/z 313.2 ($MH^+$), 255.1, 201.1, 185.1, 168.1, 111.1. Microanalysis calculated for $C_{16}H_{24}O_6$: C 61.51, H 7.74. Found C 61.47, H 7.81. $[\alpha]_D^{28}$ -62.5 ($CHCl_3$).

Example 8

**(6*R*,7*R*,15*S*)-15-(1-hydroxycyclopentan-1-yl)-15-methyl-1,8,13,16-tetraoxadispiro[5.0.5.4]-hexadecan-14-one, 9d.**

The standard procedure using **3** (302 mg, 1.18 mmol) and cyclopentanone (209 μl, 2 eq.) gave, in order of elution, starting material **3** (139 mg, 46%) and compound **9d** (142 mg, 35%) after flash chromatography (20% ether/hexane).

**9d**: MPt. 131-133°C (60-80 petrol). $^1$H NMR ($CDCl_3$, 400 MHz) δ 3.95-3.85 (1H, m), 3.83-3.75 (1H, m), 3.73-3.63 (3H, m, incl. 3.73 1H, s, OH; 2×H2, 2×H9), 1.99-1.87 (4H, m), 1.80-1.47 (15H, m, incl. 1.52, 3H, s, Me; 8×$CH_2$); $^{13}$C NMR ($CDCl_3$, 100 MHz) δ 171.3 (C=O), 103.6, 95.6 (C6, C7), 87.0, 81.1 (C15, C1'), 62.6, 62.2 (C2, C9), 36.0, 34.4, 29.1, 28.7, 24.9, 24.5, 24.2, 23.8 (8×$CH_2$), 23.2 (Me), 18.3, 17.3 (2×$CH_2$); IR (thin film) 3497, 2950, 1745, 1289, 1213, 1073, 983 $cm^{-1}$; MS (EI) m/z 341.2 ($MH^+$), 256.1, 201.1, 185.1, 168.1, 140.1, 122.1, 111.1, 101.1. Analysis calculated for $C_{18}H_{28}O_6$: C 63.51, H 8.29. Found C 63.62, H 8.31. $[\alpha]_D^{29}$ -61.5 (c=1.18, $CHCl_3$).

Example 9

**[6*S*,7*R*,15*S*,(*S*)]-15-(1-hydroxy-1-phenylethyl)-15-methyl-1,8,13,16-tetraoxadispiro[5.0.5.4]-hexadecan-14-one, 9e.**

The standard procedure using **3** (306 mg, 1.2 mmol) and acetophenone (279 μl, 2.0 eq.) gave, in order of elution, starting material **3** (170 mg, 56%) and **9e** (130 mg, 29%) after flash chromatography (10% EtOAc/hexane).

**9e**: MPt. 143-144°C (ether/$CHCl_3$); $^1$H NMR ($CDCl_3$, 400 MHz) δ 7.56 (2H, dd, J=1.2, 7.7 Hz, *ortho*-H), 7.33-7.24 (3H, m, *meta/para*-H), 4.54 (1H, s, OH), 4.07-3.97 (1H, m), 3.80 (1H, dd, J=1.6, 10.4 Hz), 3.68-3.58 (2H, m, 2×H2, 2×H9), 2.06-1.93 (3H, m), 1.74-1.44 (15H, m, incl. 1.67, 3H, s, Me2' and 1.44, 3H, s, Me; 6×$CH_2$); $^{13}$C NMR ($CDCl_3$, 100 MHz) δ 172.1 (C=O), 142.0 (*ipso*-C), 127.6, 126.9 (*ortho/meta*-C; *para*-C probably coincident at δ 126.9), 104.1, 95.6 (C6, C7), 80.5, 77.9 (C15, C1'), 62.5, 62.0 (C2, C9), 29.2, 28.6 (2×$CH_2$), 25.8 (Me), 24.7, 24.2 (2×$CH_2$), 23.2 (Me), 18.1, 17.3 (2×$CH_2$); IR (thin film) 3477, 2951, 2888, 1753, 1713, 1447, 1363, 1273, 1215, 1102, 1074, 1002 $cm^{-1}$; MS (EI) m/z 377.2 ($MH^+$), 359.2, 312.2, 257.1, 183.1, 168.1. Accurate mass calculated for $C_{21}H_{27}O_5$ $(M-OH)^+$: 359.1858. Found 359.1877. $[\alpha]_D^{29}$ -75.4 (c=1.21, $CHCl_3$).

## SCHEME 1

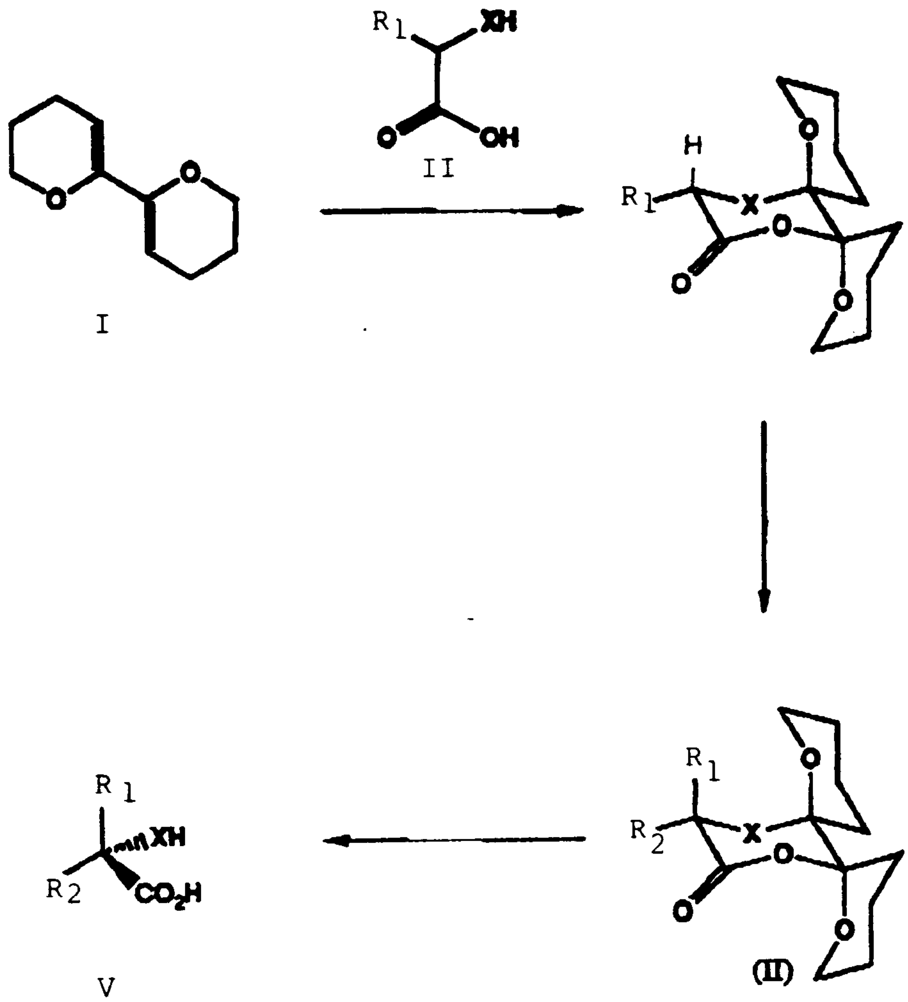

I

II

III

IV

(II)

V

## SCHEME 2

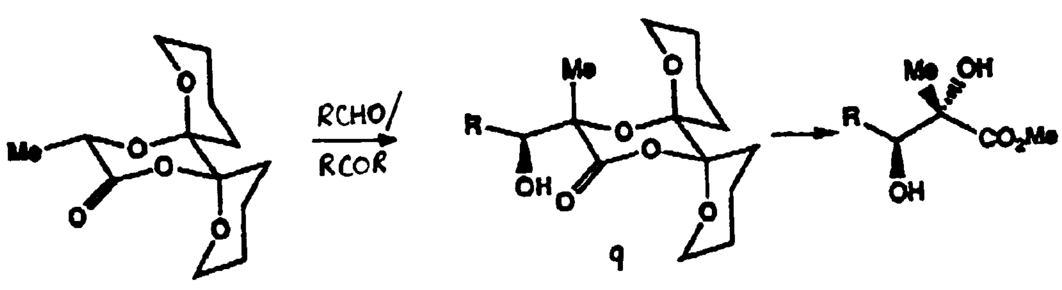


9

SCHEME 3

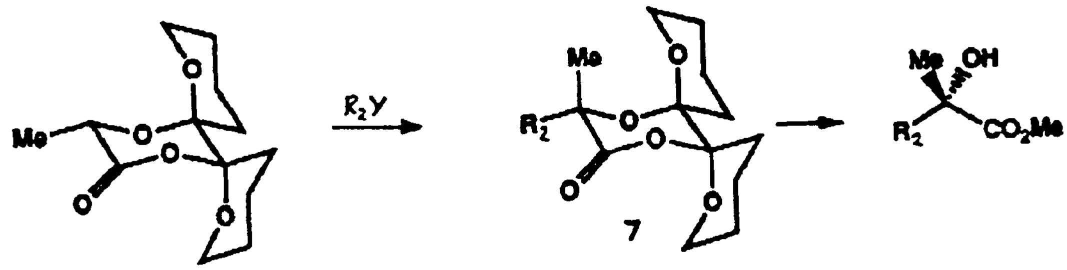


7

SCHEME 4

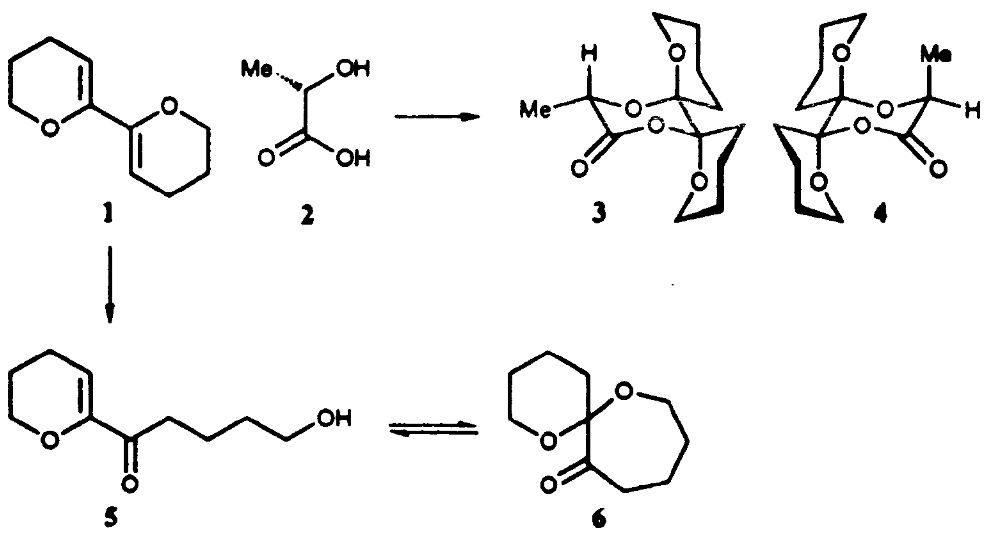

1 2 3 4 5 6

## Claims

1. An adduct of a 2,2'-bis(cyclic vinyl ether) and a chiral compound that has the formula

$$R_1CH(XH)\text{-}COOH$$

wherein $R_1$ is an inert substituent and X is a heteroatom.

2. An adduct according to claim 1, wherein X is -O-.

3. An adduct according to claim 2, wherein the bis-cyclic vinyl ether is bis-hydropyran.

4. A process for preparing an adduct according to any of claims 1 to 3, which comprises reaction of the bis-cyclic vinyl ether and the chiral compound (if necessary, in the presence of an additional acid catalyst) and in the absence of water.

5. A process for preparing an acid of the formula

$$R_1R_2C(XH)\text{-}COOH$$

in enantiopure form, which comprises reaction of an adduct according to any of claims 1 to 3, with base

and then with a corresponding electrophile capable of introducing the group $R_2$, and releasing the desired enantiomer from the appropriate diastereomer.

European Patent Office

# EUROPEAN SEARCH REPORT

Application Number
EP 94 30 6404

| Category | Citation of document with indication, where appropriate, of relevant passages | Relevant to claim | CLASSIFICATION OF THE APPLICATION (Int.Cl.6) |
|---|---|---|---|
| D,A | SYNTHESIS, no.1/2, 1992, STUTTGART DE pages 52 - 54 S.V. LEY ET AL. 'Dispiroketals in synthesis: preparation of a stable, sterically demanding glyceraldehyde ketal and diastereoselective reactions with simple organometallic reagents' * the whole document * | 1-5 | C07D493/20<br>C07C51/353<br>C07C59/01<br>//(C07D493/20, 319:00,311:00, 311:00) |
| D,A | TETRAHEDRON, vol.40, no.8, 1984, OXFORD GB pages 1313 - 1324 D. SEEBACH ET AL. 'Alpha-alkylation of alpha-heterosubstituted carboxylic acids without racemization' * the whole document * | 1-5 | |
| D,A | TETRAHEDRON LETTERS, vol.33, no.33, 11 August 1992, OXFORD GB pages 4767 - 4770 S.V. LEY ET AL. 'Dispiroketals in synthesis (part 2): a new group for selective protection of diequatorial vicinal diols in carbohydrates' * the whole document * | 1-5 | TECHNICAL FIELDS SEARCHED (Int.Cl.6)<br>C07D |
| P,X | TETRAHEDRON LETTERS, vol.35, no.5, 31 January 1994, OXFORD GB pages 769 - 772 R. DOWNHAM ET AL. 'Dispiroketals in synthesis (part 6): highly stereoselective alkylation of dispiroketal protected lactate and glycolate enolates' * the whole document * | 1-5 | |

The present search report has been drawn up for all claims

| Place of search | Date of completion of the search | Examiner |
|---|---|---|
| THE HAGUE | 25 October 1994 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)